# FASCICULE DE BREVET EUROPEEN

(11) **EP 2 804 628 B1**
(45) Date de publication et mention de la délivrance du brevet: **01.03.2017**
(21) Numéro de dépôt: 13704171.1
(22) Date de dépôt: 17.01.2013
(51) Int. Cl.: A61K 39/295, A61P 31/04, A61P 31/12

(54) **PROCÉDÉ DE FORMULATION D'UN VACCIN CONTENANT AU MOINS DEUX ANTIGÈNES SUSCEPTIBLES DE S'ADSORBER SUR DE L'OXYHYDROXYDE D'ALUMINIUM**
VERFAHREN ZUR FORMULIERUNG EINES IMPFSTOFFES MIT MINDESTENS ZWEI ANTIGENEN ZUR ADSORPTION AUF ALUMINIUMOXIHYDROXID
METHOD FOR FORMULATING A VACCINE CONTAINING AT LEAST TWO ANTIGENS CAPABLE OF ADSORBING ONTO ALUMINIUM OXYHYDROXIDE

(30) Priorité: 17.01.2012 FR 1250464
(43) Date de publication de la demande: 26.11.2014
(73) Titulaire: Sanofi Pasteur, 69367 Lyon Cedex 07 (FR)
(72) Inventeur: BERTAUX, Landry, F-69009 Lyon (FR); CHACORNAC, Isabelle, F-69420 Tupin et Semons (FR); FRANÇON, Alain, F-69690 Bessenay (FR); HAU, Jean-François, F-76000 Rouen (FR); LENTSCH GRAF, Sandrine, F-69110 Sainte Foy les Lyon (FR)
(74) Mandataire: Kerneis, Daniéle
(86) Numéro de dépôt international: PCT/FR2013/050106
(87) Numéro de publication internationale: WO 2013/107988

(56) Documents cités:
- EP-A1- 1 416 958
- WO-A1-99/13906
- WO-A1-03/009869
- STURGESS A W ET AL: "Haemophilus influenzae type b conjugate vaccine stability: catalytic depolymerization of PRP in the presence of aluminum hydroxide", VACCINE, ELSEVIER LTD, GB, vol. 17, no. 9-10, 5 mars 1999 (1999-03-05), pages 1169-1178, XP004158240, ISSN: 0264-410X, DOI: 10.1016/S0264-410X(98)00337-5

## Description

L'invention est relative au domaine des combinaisons vaccinales comprenant à la fois la valence Hépatite B constituée par l'antigène de surface du virus de l'hépatite B (HBsAg) et la valence *Haemophilus influenzae* type b, constituée par son polysaccharide capsulaire, appelé polyribosylribitolphosphate (PRP) qui est, pour être efficace chez les enfants de moins dé deux ans, conjugué à une protéine porteuse, par exemple la protéine tétanique.

De telles combinaisons qui sont destinées à l'administration des enfants en bas âge, comprennent généralement d'autres antigènes permettant de vacciner, en une seule opération, contre plusieurs maladies, ainsi qu'un adjuvant à base d'aluminium.

La demande WO 03/009869 s'intéresse au problème de la stabilité des vaccins et recommande, lorsque l'antigène de l'hépatite B est présent, d'éviter de l'adsorber sur hydroxyde d'aluminium ; il est conseillé de l'adsorber sur de l'hydroxyphosphate d'aluminium ou de ne pas l'adsorber.

La demande de brevet WO99/13906 divulgue une composition vaccinale comprenant, ainsi que cela est décrit à la page 13, des antigènes contre la diphtérie, le tétanos, la polio, la coqueluche, l'hépatite B et les infections à *Haemophilus influenzae* type b. Certains de ces antigènes ont besoin, pour être immunogènes, d'être adsorbés sur un sel d'aluminium. C'est le cas notamment de l'antigène de surface de l'hépatite B ou HBsAg.

Or, ainsi que cela est indiqué à la page 12 de la demande WO99/13906, la valence Hib constituée par le polysaccharide capsulaire conjugué à la protéine tétanique, a tendance à perdre de son immunogénicité au cours du temps lorsqu'elle est adsorbée sur des sels d'aluminium. Pour éviter cet inconvénient, la solution proposée dans cet art antérieur est, ainsi que cela avait déjà été recommandé dans la demande antérieure PCT/FR96/00791, d'ajouter des anions et notamment des ions phosphate, carbonate, ou citrate.

Cependant, les auteurs de la présente invention ont remarqué que si l'ajout d'anions, notamment de phosphates ou de carbonates, permet effectivement d'éviter l'adsorption du PRP-T sur l'oxyhydroxyde d'aluminium (AlOOH), et donc de maintenir son immunogénicité au cours du temps, cet ajout a aussi pour inconvénient dé désorber l'antigène de surface de l'hépatite B lorsque celui-ci avait été, lui aussi, adsorbé sur de l'oxyhydroxyde d'aluminium.

Il est donc nécessaire de trouver un procédé de préparation d'une combinaison vaccinale comprenant de l'oxyhydroxyde d'aluminium, dans laquelle l'antigène de surface de l'hépatite B est maintenu adsorbé sur AlOOH alors que l'antigène de Hib est maintenu non adsorbé.

A cette fin, la présente invention a pour objet un procédé de préparation d'une combinaison vaccinale liquide comprenant au moins :
- un antigène de surface de l'hépatite B (HBsAg),
- un antigène de *Haemophilus influenzae* type b (Hib) constitué par du polysaccharide capsulaire conjugué à une protéine porteuse,
- de l'oxyhydroxyde d'aluminium (AlOOH),
dans laquelle l'antigène de surface de l'hépatite B est maintenu adsorbé sur AlOOH alors que l'antigène de Hib est maintenu non adsorbé,
selon lequel :
- on procède à l'adsorption de l'antigène de surface de l'hépatite B sur AlOOH afin d'obtenir un complexe AlOOH/HBsAg,
- on mélange ledit complexe AlOOH/HBsAg avec l'antigène de Hib en présence d'acides aminés cationiques à une concentration d'au moins 100mg/l, et d'ions Phosphate à une concentration de 35 à 45 mMol/l.

Grâce au procédé selon l'invention, il est possible d'atteindre l'équilibre voulu entre le maintien de l'adsorption de l'antigène de surface de l'hépatite B sur l'oxyhydroxyde d'aluminium et le maintien de la non-adsorption de l'antigène de Hib.

Selon un mode particulier de réalisation de l'invention, on procède à l'adsorption de l'antigène HBsAg sur l'aluminium en mélangeant une suspension d'AlOOH à une suspension d'HBsAg sous agitation pendant au moins 4 heures, de préférence au moins 12 heures, de préférence entre 20 et 24 heures.

Selon un mode particulier de réalisation de l'invention, on ajoute les acides aminés cationiques audit complexe AlOOH/HBsAg avant le mélange avec l'antigène de Hib.

Selon un mode alternatif de réalisation de l'invention, on ajoute les acides aminés cationiques audit antigène de Hib avant le mélange avec le complexe AlOOH/HBsAg. Selon un mode de réalisation de l'invention, on ajoute les ions Phosphate audit complexe AlOOH/HBsAg avant le mélange avec l'antigène Hib.

Selon un mode de réalisation de l'invention, on ajuste le pH de la préparation comprenant le complexe AlOOH/HBsAg à 7.1 ± 0.1 avant le mélange avec l'antigène de Hib.

L'invention a également pour objet une combinaison vaccinale obtenue selon le procédé revendiqué et comprenant au moins :
- l'antigène de surface de l'hépatite B,
- l'antigène de la diphtérie sous forme de toxine diphtérique D,
- l'antigène du tétanos sous forme de toxine tétanique T,
- les antigènes de la coqueluche sous forme de Toxine Purifiée (PTxd) et d'Haemagglutinine Filamenteuse (FHA),
- l'antigène de *Haemophilus influenzae* type b, sous forme de polyribosylribitolphosphate conjugué à la protéine tétanique (PRP-T),
- les antigènes de la polio sous forme de virus inactivés de type 1, 2 et 3.

Une telle composition vaccinale présente l'avantage de se présenter sous forme liquide, ce qui évite des opérations de reprise de lyophilisat; elle s'est montrée suffisamment stable pour rester immunogène jusqu'au jour de son utilisation, et ce, même à 36 mois après sa date de fabrication.

Selon l'invention, la composition vaccinale comprend un antigène de surface de l'hépatite B (HBsAg). Cet antigène peut notamment être un antigène de surface de l'hépatite B tel que celui présent dans le vaccin Recombivax HB™, ou dans tout autre vaccin contre l'hépatite B. Il peut notamment s'agir de l'antigène recombinant obtenu par fermentation d'une levure *Hansenula polymorpha* modifiée, selon la technologie développée par Crucell, tel que celui présent dans le vaccin Hepavax-Gene™. Aux fins de l'invention, la quantité d' HBsAg présent dans une dose de 0.5 ml est avantageusement comprise entre 5 et 15 µg, et en particulier de 10 µg.

Selon l'invention, la composition vaccinale comprend un antigène de *Haemophilus influenzae* type b (Hib). Cet antigène est constitué par le polysaccharide capsulaire de la bactérie ou PolyribosylRibitolPhosphate (PRP) qui est conjugué à une protéine porteuse.

La protéine porteuse peut être n'importe quelle protéine en usage à ce titre dans le domaine des vaccins. Ce peut être par exemple la toxine diphtérique D, la toxine tétanique T, la lipoprotéine D d'*Haemophilus influenzae,* la CRM197 ou la protéine de membrane externe (OMP) de *N. meningitidis.* On utilise de préférence la protéine tétanique afin d'obtenir le conjugué PRP-T. Aux fins de la présente invention, le conjugué PRP-T peut être présent à raison de 1 à 30 µg de PRP par dose de 0.5 ml; avantageusement de 5 à 25 µg de PRP par dose ; de préférence de 10 à 15 µg de PRP par dose ; de manière tout à fait préférée de 10 à 12 µg de PRP par dose, et plus particulièrement 12 µg de PRP, conjugué à 22-36 µg de protéine tétanique.

Selon l'invention, la composition vaccinale comprend de l'oxyhydroxyde d'Aluminium AlOOH. Ce sel d'aluminium est très souvent appelé par abus de langage hydroxyde d'aluminium. L'AlOOH pouvant être utilisé aux fins de la présente invention peut être par exemple l'AlOOH commercialisé par Brenntag AG ou le Rehydragel HPA de Reheis Corp. (Berkeley Heights, NJ), bien que le mode de production de chacun des deux adjuvants diffère. La quantité d'AlOOH utilisée est calculée pour permettre l'atteinte d'une réponse immunitaire satisfaisante ; elle dépend notamment du nombre et de la nature des antigènes présents dans la composition ainsi que de la quantité de chacun de ces antigènes.

A titre informatif seulement, le maximum d'adsorption de l'HBsAg sur l'AlOOH est de l'ordre de 780 µg de protéine par mg d'aluminium (de manière classique la quantité d'AlOOH est exprimée en quantité d'aluminium Al³⁺). Pour une dose vaccinale comprenant 10 µg d'HBsAg, sans autres antigènes additionnels, il suffirait donc de 13 µg d'aluminium, quantité toutefois insuffisante pour obtenir l'efficacité recherchée lorsque d'autres antigènes sont rajoutés. Ainsi, en fonction de l'ajout d'un ou plusieurs antigènes supplémentaires, on peut mettre en contact 10 µg d'HBsAg avec de 0.01 mg à 1.25 mg d'aluminium qui est la quantité maximale recommandée par la Pharmacopée européenne.

Par exemple, une dose de 0.5 ml d'un vaccin pédiatrique comprenant des antigènes de la diphtérie, du tétanos, de la coqueluche, de l'hépatite B, peut contenir de manière conventionnelle de 0.5 à 0.7 mg d'aluminium, de préférence environ 0.6 mg d'aluminium.

Selon l'invention, l'antigène de surface de l'hépatite B est maintenu adsorbé sur AlOOH, ce qui signifie qu'au moins 60%, de préférence au moins 65%, de préférence au moins 70%, de préférence au moins 75%, de préférence au moins 80%, de préférence au moins 85%, de préférence au moins 90%, de préférence au moins 95% de la quantité totale de cet antigène présent dans la composition l'est sous forme adsorbée.

Selon l'invention, l'antigène de Hib est maintenu non adsorbé sur AlOOH, ce qui signifie qu'au moins 65 %, de préférence au moins 70 %, de préférence au moins 75 %, de la quantité totale de cet antigène présent dans la composition l'est sous forme non-adsorbée.

Selon l'invention, la durée pendant laquelle l'antigène de surface de l'hépatite B est maintenu adsorbé sur l'AlOOH et l'antigène de Hib est maintenu non adsorbé est d'au moins 3 mois, de préférence au moins 6 mois, de préférence au moins 12 mois, de préférence au moins 18 mois; de préférence au moins 24 mois, de préférence encore au moins 36 mois à partir de la date de fabrication de la composition quand la température de conservation est de 5 ± 3°C. De préférence, la quantité d'antigènes adsorbés ou non-adsorbés est stable au cours du temps, mais elle peut également varier, à condition de rester dans des limites acceptables. Ainsi, lorsqu'au moins 85 % de la quantité totale de l'HBsAg présente dans la composition est adsorbé sur l'AlOOH pendant 3 mois à partir de la date de fabrication de la composition conservée à une température de 5 ± 3°C, il est tout à fait possible qu'au bout d'un an et toujours dans les mêmes conditions de conservation, 80 % de la quantité totale de l'HBsAg présente dans la composition soit maintenu adsorbé.

Pour apprécier la quantité d'antigène adsorbé, l'homme du métier peut utiliser toute méthode connue.

En ce qui concerne la détermination du pourcentage d'adsorption de l'HBsAg, il est possible d'utiliser une méthode ELISA de type sandwich selon les règles définies par la Pharmacopée Européenne 2.7.1. Brièvement, l'HBsAg est capturé par un anticorps monoclonal primaire anti-HBsAg de type IgM, dans des puits d'une plaque de 96 puits. L'HBsAg ainsi fixé est recouvert par un anticorps monoclonal secondaire anti-HBsAg, de type IgG, qui est lui-même détecté par un anticorps polyclonal anti-IgG, couplé à la peroxydase. Un substrat chromogène de la peroxydase, la tétraméthylbenzidine (TMB), sert d'agent révélateur. Lors de son ajout une couleur se développe dont l'intensité est proportionnelle à la quantité d'HBsAg capturé dans le puits. L'analyse des résultats s'effectue selon la méthode des droites parallèles décrite dans la Pharmacopée Européenne 5.3.3. Le pourcentage d'adsorption est obtenu à partir de la détermination du contenu total en HBsAg et du contenu en HBsAg non-adsorbé.

En ce qui concerne la quantité de PRP-T non adsorbée, on peut faire l'évaluation par HPAEC-PAD (chromatographie d'échange d'ions haute performance - détection ampérométrique pulsée).

Selon le procédé de l'invention, l'antigène de surface de l'hépatite B est adsorbé sur l'AlOOH. Cette étape peut être réalisée en mettant en contact l'antigène de surface de l'hépatite B et l'AlOOH en absence de tout autre antigène et en laissant l'antigène de surface de l'hépatite B s'adsorber sur l'AlOOH pendant au moins 4 heures, de préférence au moins 12 heures, de manière tout à fait préférée entre 20 et 24 heures, pour obtenir une préparation contenant un complexe AlOOH/HBsAg. Cette adsorption peut être réalisée, selon l'invention, en absence d'ions phosphate. L'objectif poursuivi par un temps de contact prolongé entre l'antigène de surface de l'hépatite B et l'AlOOH consiste à maximiser les interactions électrostatiques et à favoriser les interactions stables, pouvant aboutir ainsi à une adsorption par échange de ligand. Ce contact est avantageusement poursuivi sous agitation.

Selon le procédé de l'invention, on réalise le mélange du complexe AlOOH/HBsAg avec l'antigène de Hib en présence d'acides aminés cationiques et d'ions Phosphate.

Aux fins de l'invention, on entend par acides aminés cationiques des acides aminés dont le pHi est supérieur au pH de la composition vaccinale et qui seront donc sous forme cationique au pH du vaccin ; il s'agit notamment de la Lysine (Lys), l'Arginine (Arg) ou l'Histidine (His) ; chacun de ces acides aminés peut être utilisé seul, ou en mélange soit par 2 (Lys + Arg ; Lys + His ; Arg + His), soit les 3 ensemble (Lys + Arg + His). Selon un mode particulier, les acides aminés cationiques peuvent être associés sous forme de dipeptide. On cite notamment les dipeptides Lys-Lys, Lys-Arg, Lys-His, Arg-Arg, Arg-Lys, Arg-His, His-His, His-Lys et His-Arg. De manière alternative, un dipeptide utile aux fins de la présente invention peut être composé d'un acide aminé cationique et d'un acide aminé non-chargé sélectionné parmi Ala, Val, Leu, Iso, Pro, Met, Phe, Trp, Gly, Ser, Thr, Cys, Tyr, Asp et Gln. Ainsi, en pratique, on peut utiliser une préparation contenant un ou plusieurs acide(s) aminé(s) cationiques sous forme libre et/ou de dipeptide. Il est également possible d'utiliser des préparations d'acides aminés comprenant à la fois des acides aminés cationiques en quantité désirée, en mélange avec d'autres acides aminés. Afin de ne pas produire une trop grande chute du pH lors de l'ajout des acides aminés, on peut prévoir d'augmenter le pH de la préparation comprenant les acides aminés avant de l'ajouter au complexe AlOOH/HBsAg, au moyen d'une base, notamment de la soude.

Selon l'invention, la quantité d'acides aminés cationiques présents dans la composition vaccinale au final, doit être d'au moins 100 mg/l, avantageusement d'au moins 300 mg/l, avantageusement d'au moins 400 mg/l ; de manière tout à fait préférée d'au moins 500 mg/l. Il n'existe pas de dose maximale critique. Cependant, il est préférable que la quantité maximale soit au plus 2 mg/ml, de préférence encore au plus 1 mg/ml; de préférence encore au plus 800 µg/ml ; de manière tout à fait préférée au plus 700µg/ml. Lors du calcul de la quantité d'acides aminés cationiques à ajouter, il convient de tenir compte des acides aminés cationiques susceptibles d'être apportés par les milieux dans lesquels sont présents les antigènes autres que l'HBsAg et l'antigène de Hib.

Selon une alternative du procédé, on peut déterminer la quantité d'acides aminés cationiques par rapport au poids du polysaccharide capsulaire de Hib et prévoir un rapport en poids polysaccharide de Hib/acide aminé cationique de 1 : 4 à 1 : 100, avantageusement de 1 : 10 à 1 : 80, de préférence de 1 : 15 à 1 : 60, de manière tout particulièrement préférée de 1 : 20 à 1 : 30 ou 40.

Selon le procédé de l'invention, le mélange du complexe AlOOH/HBsAg avec l'antigène de Hib est réalisé en présence d'acides aminés cationiques, mais également en présence d'ions Phosphate. Selon un mode de réalisation de l'invention, les ions Phosphates sont ajoutés au complexe AlOOH/HBsAg avant sa mise en contact avec l'antigène Hib. L'apport d'ions Phosphate peut par exemple être réalisé par ajout d'hydrogenophosphate de sodium ou d'hydrogenophosphate de potassium, ou encore par un mélange des 2. La quantité d'ions phosphate est calculée pour que le maximum d'antigènes de surface de l'hépatite B reste adsorbé sur l'AlOOH tout en évitant l'adsorption de l'antigène Hib. Cette quantité varie en fonction de la nature et du nombre des antigènes présents, et notamment en fonction des antigènes autres que les antigènes HBsAg et Hib.

Ainsi, pour une combinaison vaccinale comprenant les antigènes habituellement utilisés dans les vaccins pédiatriques, soient en plus de l'HBsAg et de l'antigène Hib, les antigènes de la diphtérie, du tétanos, de la coqueluche, il peut être avantageux d'ajouter des ions phosphate de telle sorte que la concentration d'ions phosphate dans la composition vaccinale obtenue au final soit au moins égale à 35 mMol/l, et plus particulièrement soit comprise entre 35 et 45 mMol/l, bornes incluses ; de préférence entre 38 et 44 mMol/l, bornes incluses ; de manière tout à fait préférée, entre 38 et 42 mMol/l, bornes incluses. Selon un mode préféré, la concentration d'ions phosphate dans la composition vaccinale obtenue en dernier lieu est de 40 mMol/l.

Selon un mode alternatif, les ions phosphate sont ajoutés en quantité telle qu'ils sont présents dans la composition vaccinale à une concentration finale comprise entre 35 et 38 mMol/l, bornes incluses. On complète en ajoutant en outre des ions carbonate, mais cependant en quantité limitée, car on a en effet remarqué qu'une trop grande quantité d'ions carbonates était défavorable. Avantageusement, ils peuvent être ajoutés en quantité telle qu'ils sont présents dans la composition vaccinale à une concentration finale inférieure ou égale à 10 mMol/l.

Afin d'éviter un choc ionique trop important qui pourrait déstabiliser l'HBsAg et favoriser sa désorption, on préconise d'ajouter les ions phosphate en plusieurs (par exemple en 2) opérations (étapes) distinctes. Ainsi, les ions phosphate peuvent être ajoutés en une première opération, en quantité permettant d'atteindre une concentration finale comprise entre 20 et 30 mMol/l bornes incluses ; puis en une deuxième opération, en quantité permettant d'atteindre une concentration finale telle que spécifiée ci-dessus.

Selon un mode de réalisation préféré du procédé selon l'invention, on ajuste en outré le pH de la préparation obtenue à 7.1 ± 0.1, avant de mélanger l'antigène Hib avec le complexe AlOOH/HBsAg. On a en effet remarqué qu'une telle valeur de pH avait un effet positif sur le maintien à l'état non-adsorbé de l'antigène Hib.

Selon un mode de réalisation particulier, on ajuste en outre le pH à 7.1 ± 0.1 après la phase de mélange.

Ainsi, grâce au procédé selon l'invention, on peut obtenir une composition vaccinale dans laquelle :
(i) au moins 60 ou 80 %, de préférence au moins 85 % de la quantité totale de l'antigène de surface de l'hépatite B présent dans la composition est adsorbé sur l'AlOOH pendant au moins 3 mois à partir de la date de fabrication de la composition conservée à une température de 5 ± 3°C ; et
(ii) au moins 65, 70 ou 75 % de la quantité totale de l'antigène Hib présent dans la composition n'est pas adsorbé sur l'AlOOH.

L'expression « complexe AlOOH/HBsAg » doit être interprétée comme signifiant que le complexe comprend au moins l'antigène HBsAg adsorbé sur l'AlOOH. Le complexe peut contenir d'autres antigènes, que cela soit précisé ou pas.

Un ou plusieurs antigènes additionnels peu(ven)t venir en outre former le complexe. Il peut notamment s'agir de l'anatoxine diphtérique (D), de l'anatoxine tétanique (T), des antigènes acellulaires de la coqueluche tels que : la toxine détoxifiée de *Bordetella pertussis* (PTxd), l'hémagglutinine filamenteuse (FHA), la pertactine (antigène de 69 kDa) et des agglutinogènes (fimbriae) de cette même bactérie. Selon un mode particulièrement avantageux, pour former le complexe, on peut ajouter des antigènes D, T, PTxd et FHA.

Les antigènes additionnels peuvent être ajoutés de diverses manières. Ils peuvent être ajoutés de manière séquentielle à la suite de l'antigène de surface de l'hépatite B adsorbé au préalable (i) soit sur la quantité totale d'AlOOH devant être présente dans la composition vaccinale ; (ii) soit sur une quantité partielle, complétée par la suite pour attendre la quantité totale. De manière alternative, les antigènes additionnels peuvent être adsorbés de manière séparée, chacun sur une quantité partielle d'AlOOH tout comme l'HBsAg. On peut aussi prévoir un processus d'adsorption mixte - certains antigènes étant adsorbés de manière séparée ; d'autres étant adsorbés de manière séquentielle.

Selon un mode particulier du procédé selon l'invention, on agite la composition obtenue après ajout de chaque antigène.

Selon un mode avantageux et donné uniquement à titre d'exemple, l'HBsAg est adsorbé de manière séparée sur une quantité partielle d'AlOOH correspondant à environ 30 % (le tiers) de la quantité totale d'AlOOH présente dans la composition finale. En parallèle, les antigènes D et T sont adsorbés de manière séquentielle sur la partie complémentaire de l'AlOOH. Puis à la préparation contenant le complexe AlOOH-D-T, on ajoute les antigènes de la coqueluche PTxd et FHA, chacun de ces 2 derniers antigènes ayant eux-mêmes été au préalable adsorbés individuellement sur de l'AlOOH. Enfin, on rassemble les deux préparations (complexe AlOOH - HBsAg et complexe AlOOH - D-T-PTxd-FHA) pour former une préparation comprenant le complexe AlOOH - HBsAg-D-T-PTxd-FHA, dans lequel les quantités de chacun des éléments ont été choisies pour obtenir des doses vaccinales de 0.5ml comprenant, de façon classique :
- de 5 à 15 µg d'HBsAg /dose ; de préférence 10 µg /dose,
- de 20 à 40 Lf de D / dose ; de préférence de 25 à 35 Lf / dose ; de manière tout à fait préférée, 30 Lf / dose. (Lf = limite de floculation), (exprimée d'une autre manière, la quantité de D est supérieure ou égale à 20 UI/dose)
- de 5 à 25 Lf de T /dose ; de préférence de 10 à 15 Lf / dose ; de manière tout à fait préférée, 10 Lf / dose, (exprimée d'une autre manière, la quantité de T est supérieure ou égale à 40 UI/ dose)
- de 20 à 30 µg de FHA / dose ; de préférence 25 µg / dose,
- de 20 à 30 µg de PTxd / dose ; de préférence 25 µg / dose.

Selon un mode particulier de réalisation de l'invention, on ajoute également des antigènes de la polio qui sont constitués, de façon conventionnelle, par des virus inactivés. On peut prévoir d'ajouter des virus de la polio des 3 types habituellement présents dans les vaccins pédiatriques, c'est-à-dire les types 1, 2 ou 3, ou bien dans le cas où il ne serait pas nécessaire de vacciner contre les 3 types, de n'introduire que les types contre lesquels la protection est recherchée. Les quantités de virus de la polio par dose peuvent notamment être :
- entre 20 et 43 DU (unités d'antigènes D), en particulier 40 pour le type 1,
- entre 5 et 9 DU, en particulier 8, pour le type 2,
- entre 17 et 36 DU, en particulier 32, pour le type 3.

Ces antigènes ne sont pas nécessairement adsorbés au préalable sur un sel d'aluminium avant d'être ajoutés à la préparation vaccinale.

Selon un mode de réalisation particulier, le procédé selon l'invention est un procédé selon lequel :
(i) (a) on met en contact l'HBsAg et l'AlOOH en l'absence de tout autre antigène, et on laisse l'HBsAg s'adsorber sur l'AlOOH pendant au moins 4 heures, de préférence au moins 12 heures, de manière tout à fait préférée environ 24 heures, pour obtenir une préparation contenant un complexe AlOOH/HBsAg ;
(i) (b) à la préparation obtenue au point (i) (a), on ajoute une préparation comprenant les antigènes D, T, PTxd et FHA, préalablement adsorbés sur AlOOH, et de manière optionnelle, des antigènes additionnels de *Bordetella.* pertussis tels que la Pertactine et les agglutinogènes ;
(ii) à la préparation obtenue au point (i) (b), on ajoute des ions phosphate afin d'obtenir une concentration finale dans le vaccin de 40mMol/l,
(iii) à la préparation obtenue au point (ii), on ajoute de manière optionnelle, les antigènes de la polio ;
(iv) à la préparation obtenue au point (ii) ou (iii), on ajoute une préparation contenant l'antigène Hib ;
(v) on ajuste le pH à 7.1 ± 0.1; et
(vi)
   (a) on ajoute au moins un acide aminé cationique pour compléter la préparation obtenue au point (ii) ou (iii) avant l'ajout de l'antigène Hib, ou
   (b) on ajoute à l'antigèné Hib au moins acide aminé cationique ;
ledit acide aminé cationique étant ajouté en quantité suffisante afin d'obtenir une concentration finale dans le vaccin d'au moins 100 mg/l.

Selon un mode particulier, la composition vaccinale selon l'invention est une composition comprenant l'HBsAg, la toxine diphtérique D, la toxine tétanique T, les antigènes de la coqueluche PTxd et FHA ayant été préadsorbés sur de l'AlOOH, l'antigène Hib, et optionnellement la valence polio, dans laquelle :
(i) au moins 85 %, de préférence au moins 90 % de la quantité totale de l'HBsAg présente dans la composition est maintenu adsorbé sur l'AlOOH pendant au moins 3 mois à partir de la date de fabrication de la composition conservée à une température de 5 ± 3°C ; et
(ii) au moins 65, 70, ou de préférence 75 % de la quantité totale de l'antigène Hib présent dans la composition n'est pas adsorbé sur l'AlOOH, cette quantité restant relativement stable au cours du temps.

En outre, grâce au procédé selon l'invention, les antigènes autres que l'HBsAg qui ont montré qu'il était avantageux qu'ils soient adsorbés sur l'oxyhydroxyde d'aluminium pour être immunogènes, sont également maintenus adsorbés.

Ainsi, on indique à titre d'exemple, qu'une composition selon l'invention peut comprendre :
- de 10 à 30 µg d'HBsAg /ml, de préférence 20 µg/ml ;
- de 40 à 80 Lf de D/ml, de préférence de 50 à 70Lf/ml;
- de 10 à 50 Lf de T/ml, de préférence de 10 à 30Lf /ml ;
- de 40 à 60 µg de FHA/ml, de préférence 50 µg/ml ;
- de 40 à 60 µg de PTxd/ml, de préférence 50 µg/ml ;
- de 2 à 60 µg de PRP/ml, de préférence 20-24 µg/ml ;
- de 1 à 2 mg d'AlOOH/ml ; de préférence 1.2 mg d'AlOOH/ml ;
- des ions phosphate à une concentration de 35 à 45 mMol/l, de préférence de 38 à 42 mMol/l d'ions phosphate ;
- de 100 à 1000 µg/ml d'acides aminés cationiques, de préférence de 400 à 800 µg/ml ; et de manière optionnelle,
- les types 1, 2 et 3 du virus polio sous forme inactivée en quantité respective de 80, 16 et 64 DU/ml.

Comme indiqué précédemment, une composition selon l'invention peut aussi comprendre des antigènes additionnels de *Bordetella. pertussis,* tels que la Pertactine(de 69kDa) ou les agglutinogènes.

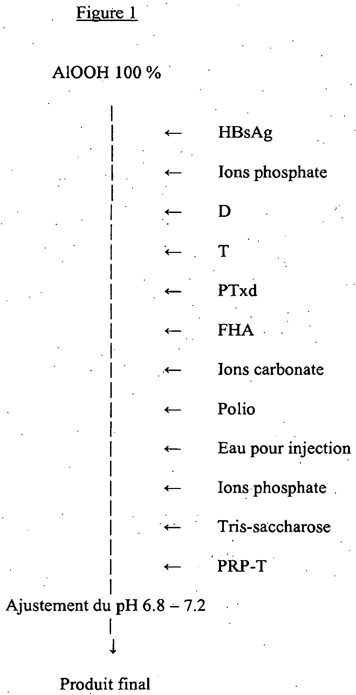

**Description de la figure :** La Figure 1 est un schéma d'un procédé de l'art antérieur selon lequel, après l'ajout de chaque composant, on mélange.

### EXEMPLE - Préparation à l'échelle industrielle d'un vrac (250 L) d'un vaccin hexavalent HepB-Dt-Tt-Pertussis-polio-HiB

Cette préparation s'effectue dans des conditions stériles et sous agitation continue.

### A - Préparation de l'HBsAg adsorbé sur AlOOH

Dans une cuve de 50 1, on introduit de manière aseptique une suspension homogène de gel d'oxyhydroxyde d'aluminium (AlOOH) commercialisé par Brenntag AG, à 8 g d'Aluminium/l.

Après filtration sur un filtre 0.22 µm, le volume nécessaire d'HBsAg, pour obtenir une concentration de 20 µg/ml dans le vaccin final, est ajouté en continu dans la cuve contenant déjà l'AlOOH.

Le mélange est laissé sous agitation pendant 20 à 24 heures à température ambiante de manière à obtenir une suspension homogène.

### B - Préparation de D + T + PTxd + FHA adsorbées sur gel d'aluminium

En parallèle, on prépare un mélange de gel d'aluminium, d'anatoxine diphtérique (D), d'anatoxine tétanique (T), d'anatoxine de *Bordetella pertussis* (PTxd) et d'hémagglutinine filamenteuse de *Bordetella*. *pertussis* (FHA) de la manière suivante :
Dans une cuve de 250 l, on introduit de manière aseptique, une suspension homogène de gel d'AlOOH commercialisé par Brenntag AG, à 8 g d'Aluminium /l.

Dans la cuve de 250 l contenant déjà l'AlOOH, sont introduits successivement, après filtration sur filtre 0.22 µm, les solutions de D puis après homogénéisation, de T de manière à obtenir les concentrations respectives en D et T dans le vaccin final de 60 Lf (limite de floculation)/ml et 20 Lf/ml.

Une fois le mélange homogène obtenu, sont ajoutés successivement dans cette cuve, de manière aseptique la suspension de PTxd préalablement adsorbée sur AlOOH, puis la suspension de FHA préalablement adsorbée sur AlOOH afin d'obtenir les concentrations en PTxd et FHA dans le vaccin final de 25 µg/ml.

Enfin, on ajoute, après filtration sur un filtre 0.22 µm, le volume nécessaire de tampon phosphate 500 mM pour obtenir une concentration en ions phosphate de 20 à 30 mMol/l.

La suspension D-T-PTxd-FHA-AlOOH ainsi obtenue est laissée sous agitation au moins 14 heures à une température de 5 ± 3°C.

### C - Préparation du mélange HBsAg + D + T + PTxd + FHA adsorbés sur gel d'aluminium

A la préparation obtenue au point B, on ajoute de manière aseptique la préparation obtenue au point A.

Ce mélange est laissé sous agitation de manière à obtenir une suspension homogène.

Puis on ajoute le volume nécessaire de tampon phosphate 500 mM après filtration sur un filtre 0.22 µm pour obtenir une concentration en ions phosphate de 40 mMol/l dans la composition finale.

### D - Saturation des sites électrostatiques du complexe gel d'aluminium / HBsAg + D+ T + PTxd + FHA par une solution d'acides aminés

On réalise une solution d'acides aminés contenant 12 acides aminés essentiels, de composition suivante :

| | |
|---|---|
| - Chlorhydrate d'Arginine | 2.1 g/l, soit 1,73 g/l d'Arginine |
| - Cystine | 1_{.}2 g/l |
| - Histidine | 0.8 g/l |
| - Isoleucine | 2.6 g/l |
| - Leucine | 2.6 g/l |
| - Chlorhydrate de Lysine | 3.65 g/l soit 2,91 g/l de Lysine |
| - Methionine | 0.75 g/l |
| - Phenylalanine | 1.65 g/l |
| - Thréonine | 2.4 g/l |
| - Tryptophane | 0.4 g/l |
| - Tyrosine | 1.8 g/l |
| - Valine | 2.35 g/l |

Soit 21,2 g/l d'acides aminés dont 5,44 g/l d'acides aminés cationiques (His - Arg-Lys).

On ajoute 450 ml soude (NaOH) 2.5 N (0.5 1 / min). On laisse l'homogénéisation se poursuivre sous agitation pendant 10 min.

Cette solution d'acides aminés, filtrée sur filtre 0.22 µm, est ajoutée en continu dans le mélange obtenu en C, de manière à obtenir une concentration de 572 µg/ml d'acides aminés cationiques dans la composition finale.

### E - Ajustement du pH

Le pH de la suspension obtenue au point D est ajusté à pH 7.1 (7.0 - 7.2) en utilisant une solution stock de soude à 2.5 N filtrée.

### F - Ajout des antigènes polio

Est alors introduite dans la cuve contenant la suspension obtenue en E, une préparation contenant les sérotypes 1, 2 et 3 du virus de la polio sous forme inactivée (souches Mahoney, MEF-1 et Saukett respectivement) filtrée sur filtre 0.22 µm.

### G - Ajout du PRP-T

On prépare tout d'abord une solution intermédiaire de PRP-T de la manière suivante : à une préparation de PRP-T filtrée sur filtre 0.22 µm est ajouté du tampon Tris saccharose préalablement filtré sur filtre 0.22 µm pour constituer un mélange intermédiaire.

Ce mélange est introduit aseptiquement dans le mélange obtenu en F.

### H - Phase finale d'ajustement

Après homogénéisation de la suspension obtenue en G, est ajoutée une quantité suffisante d'eau PPI filtrée au préalable de manière à atteindre le volume cible de 250 l. Puis, si nécessaire, le pH du mélange est ajusté à pH 7.1 ± 0.1 en ajoutant une solution de soude 2.5 N ou d'acide acétique 10 % filtrée au préalable.

Le mélange est stocké à 5 ± 3°C ; puis réparti en seringues ou en flacons à raison de 0.5 ml/dose.

Une dose de 0.5 ml contient ainsi 600 µg d'Al³⁺,10 µg d'HBsAg, pas moins de 20 IU de D, pas moins de 40 IU de T, 25 µg de Pt, 25 µg de FHA, entre 20 et 43 DU (unité d'antigène D) de polio type1, entre 5 et 9 DU de polio type 2, entre 17 et 36 DU de polio type 3, 12 µg de PRP (sous forme de PRP-T), des ions phosphate à une concentration de 40mMol/l, du tampon Tris saccharose à une concentration de 2.5 mMol/l de Tris et de 2.125 % de saccharose, ainsi que 286 µg d'acides aminés cationiques (His - Arg - Lys).

### Essai clinique

La composition vaccinale préparée selon l'exemple ci-dessus a été testée dans un essai clinique, comparativement à un vaccin hexavalent déjà présent sur le marché, l'Infanrix Hexa™ , qui permet de vacciner des enfants contre les mêmes maladies que le vaccin préparé selon l'invention (la diphtérie, le tétanos, la coqueluche, la polio, les infections à Hib et l'hépatite B), mais qui présente notamment l'inconvénient, d'avoir une partie lyophilisée, et donc de nécessiter une opération de reprise de lyophilisat préalablement à l'acte d'administration.

Lors de l'essai clinique, les 2 types de compositions vaccinales ont été administrés à des enfants, dans un schéma vaccinal comprenant 3 doses administrées à 2, 4 et 6 mois. Le vaccin liquide préparé selon le procédé de l'invention s'est montré très bien toléré, et aussi immunogène que le vaccin présent sur le marché.

### DONNEES EXPERIMENTALES

### Pourcentage d'adsorption de l'HBsAg / Quantité de PRP-T non-adsorbé

Trois lots de produit final vrac (PFV39-41-42) ainsi que trois lots répartis en flaçons (S12-13-14) - tous les lots ayant été obtenus selon l'exemple fourni - ont été conservés à + 5°C et analysés à différents temps sur une période de 9 et 22 mois respectivement. L'analyse a porté sur le pourcentage d'adsorption de l'HBsAg et la quantité de PRP-T non-adsorbé.

La détérmination du pourcentage d'adsorption de l'HBsAg a été réalisée, ainsi que cela a été indiqué précédemment à partir de la détermination du contenu total en HBsAg et du contenu en HBsAg non adsorbé, la détermination en HBsAg étant réalisée grâce à une méthode ELISA de type sandwich selon les règles définies par la Pharmacopée Européenne 2.7.1. Brièvement, l'HBsAg a été capturé par un anticorps monoclonal primaire anti-HBsAg de type IgM, dans des puits d'une plaque de 96 puits. L'HBsAg ainsi fixé a été recouvert par un anticorps monoclonal secondaire anti-HBsAg, de type IgG, qui a lui-même été détecté par un anticorps polyclonal anti-IgG, couplé à la peroxydase. Un substrat chromogène de la peroxydase, la tétraméthylbenzidine (TMB), a servi d'agent révélateur. Lors de son ajout une couleur s'est développée dont l'intensité était proportionnelle à la quantité d'HBsAg capturé dans le puits. L'analyse des résultats s'est effectuée selon la méthode des droites parallèles décrite dans la Pharmacopée Européenne 5.3.3.

Afin de déterminer le pourcentage d'adsorption de l'HBsAg, on a soumis le vaccin à une centrifugation (8800 g; 5 min; 20°C), ce qui a permis de récupérer le surnageant contenant l'HBsAg non-adsorbé. Les échantillons de surnageants à tester ont été dilués en tampon ELISA comprenant un tampon de désorption en série de 2 dans une gamme comprise par exemple entre 1/400 et 1/12800.

Les échantillons de vaccin total et de gamme standard ont été dilués en tampon ELISA comprenant un tampon de désorption, en série de 2 dans une gamme comprise par exemple entre 1/800 et 1/25600.

Une plaque de 96 puits recouverts de l'anticorps monoclonal primaire a été incubée 12 h à 5°C puis lavée avec une solution PBS-Tween 20. Les dilutions des surnageants, du vaccin total et de la gamme standard ont été réparties dans les puits. Puis on a ajouté l'anticorps secondaire et on a révélé avec l'anticorps conjugué à la peroxydase et la TMB(tetraméthylbenzidine). La réaction a été arrêtée en ajoutant de l'HCl 1 N. Après chaque étape, la plaque a été incubée 30 min à 25°C puis ensuite lavée avec la solution PBS-Tween 20. Un blanc (tampon de dilution) a été ajouté dans les puits disponibles et a subi les mêmes traitements. La plaque a été lue à DO 450 et 630 nm.

On a évalué la quantité de PRP-T non-adsorbée par HPAEC-PAD (chromatographie d'échange d'ions haute performance - détection ampérométrique pulsée) de la manière suivante :
On a préparé tout d'abord une gamme standard de PRP-T de référence de 0.5 à 12. 5 µg/ml.

On a centrifugé les échantillons à tester ainsi que les échantillons de la gamme standard à 5000 g pendant 5 min à température ambiante. Les surnageants ont été recueillis puis hydrolysés avec une solution de NaCl 1.5 N contenant de la glucosamine-1-phosphate comme standard interne. On a rajouté un blanc (NaCl 0.9 % ; NaOH 1.5 N + standard interne).

La chromatographie a été mise en oeuvre avec une phase mobile composée de NaOH 35 mM et d'acétate de sodium 114 mM injectée dans la colonne à raison de 1.2 ml / min.

On a calculé la concentration en PRP non-adsorbé (µg/ml) à partir de l'égalité suivante :
(Surface du pic PRP / surface du pic standard interne) = a x [concentration en PRP] + b dans laquelle « a » est la pente et « b » est l'ordonnée à l'origine, a et b ayant été déterminés à partir de la droite de régression.

Les résultats sont présentés dans les 4 tableaux ci-dessous :

| Pourcentage d'adsorption de l'HBsAg dans la formulation du produit final vrac (PFV 39-41-42) à + 5°C | | | |
|---|---|---|---|
| Temps écoulé après l'opération de formulation | PFV39 | PFV41 | PFV42 |
| 0 | 95 | 97 | 98 |
| 1 mois | 92 | 93 | 95 |
| 2 mois | 92 | 92 | 93 |
| 3 mois | 91 | 90 | 91 |
| 4 mois | 89 | 90 | 92 |
| 5 mois | 85 | 88 | 91 |
| 6 mois | 89 | 88 | 89 |
| 9 mois | 88 | 86 | 91 |

| Pourcentage d'adsorption de l'HBsAg dans la formulation conservée en flacons (lots S12-S13-S14) à + 5°C | | | |
|---|---|---|---|
| Temps écoulé après l'opération de formulation | S12 | S13 | S14 |
| 0 | 95 | 97 | 98 |
| 4 mois | 88 | 88 | 90 |
| 5 mois | 85 | 86 | 88 |
| 7 mois | 85 | 80 | 86 |
| 10 mois | 83 | 84 | 87 |
| 13 mois | 82 | 86 | 84 |
| 16 mois | 81 | 83 | 85 |
| 22 mois | 83 | 78 | 86 |

| PRP-T non-adsorbé (µg/ml) dans la formulation du produit final vrac (PFV 39-41-42) à + 5°C | | | |
|---|---|---|---|
| Temps écoulé après l'opération de formulation | PFV39 | PFV41 | PFV42 |
| 0 | 22.6 | 20.0 | 21.0 |
| 1 mois | 23.1 | 19.5 | 20.4 |
| 2 mois | 23.3 | 21.6 | 22.0 |
| 3 mois | 24.9 | 22.6 | 23.1 |
| 4 mois | 24.2 | 22.0 | 20.7 |
| 5 mois | 22.2 | 22.7 | 24.5 |
| 6 mois | 27.7 | 22.9 | 21.6 |
| 9 mois | 27.0 | 24.8 | 23 |

| PRP-T non-adsorbé (µg/ml) dans la formulation conservée en flacons (lots S12-S13-S14) à + 5°C | | | |
|---|---|---|---|
| Temps écoulé après l'opération de formulation | S12 | S13 | S14 |
| 0 | 22.6 | 20.0 | 21.0 |
| 4 mois | 21.1 | 21.1 | 19.8 |
| 5 mois | 23.0 | 19.6 | 18.6 |
| 7 mois | 23.1 | 21.0 | 19.5 |
| 10 mois | 25.0 | 23.4 | 21.8 |
| 13 mois | 24.6 | 22.6 | 20.8 |
| 16 mois | 23.5 | 22.3 | 20.8 |
| 22 mois | 23.9 | 22.0 | 19.9 |

Pour comparaison, on a aussi testé l'adsorption au cours du temps de l'HBsAg contenu dans trois lots de produit final vrac (FDN5-6-7) ainsi que dans trois lots répartis en flacons (S44-45-46) d'une préparation liquide composée des mêmes antigènes mais obtenue selon un procédé de formulation linéaire décrit à la Figure 1 et donc différent de celui décrit dans l'exemple ci-dessus. Cette préparation contenait dans 0.5 ml : 10 µg d'HBsAg, 30 Lf de Dt, 10 Lf de Tt, 25 µg de Pt, 25 µg de FHA, 40 DU de virus polio type 1, 8 DU de virus polio type 2, 32 DU de virus polio type 3, 12 µg de PRP (sous forme de PRP-T), 0.6 mg d'Al, des ions phosphate à une concentration de 55 mMol/l, des ions carbonate à une concentration de 20 mMol/l, du tampon Tris saccharose à une concentration de 2.5 mMol/l en Tris et 2.125 % en saccharose, et 14 µg d'acides aminés cationiques (His - Arg - Lys) provenant du milieu M 199 (valence polio), pH 6.8 - 7.2.

Les résultats obtenus étaient les suivants :

| Pourcentage d'adsorption de l'HBsAg dans la formulation du produit final vrac (FDN5-6-7) à + 5°C | | | |
|---|---|---|---|
| Temps écoulé après l'opération de formulation | FDN5 | FDN6 | FDN7 |
| 0 | 81 | 85 | 81 |
| 1 mois | 78 | 82 | 62 |
| 2 mois | 74 | 78 | 63 |
| 3 mois | 66 | 84 | 62 |
| 6 mois | 61 | 77 | 61 |

| Pourcentage d'adsorption de l'HBsAg dans la formulation conservée en flacons (lots S44-S45-S46) à + 5°C | | | |
|---|---|---|---|
| Temps écoulé après l'opération de formulation | S44 | S45 | S46 |
| 0 | 81 | 85 | 81 |
| 7 mois | 63 | 64 | 77 |
| 10 mois | 57 | 65 | 51 |
| 13 mois | 47 | 54 | 53 |
| 16 mois | 33 | 56 | 38 |
| 22 mois | 44 | 47 | 36 |
| 28 mois | 44 | 51 | 44 |
| 34 mois | 45 | 54 | 42 |
| 40 mois | 49 | 52 | 48 |

Les quantités de PRP-T non adsorbé mesurées sur la même période, ont montré qu'il y avait peu de variation par rapport au temps 0, et étaient donc satisfaisants. Cependant, ces résultats montrent que dans ce cas qui ne correspond pas à une formulation obtenue grâce à un procédé selon l'invention, l'antigène de surface de l'hépatite B ne restait pas adsorbé sur l'oxyhydroxyde d'aluminium.

### Données expérimentales portant sur les acides aminés cationiques

Une composition selon l'invention relevant directement du protocole expérimental mis en oeuvre dans l'exemple fourni et une composition obtenue grâce à un protocole modifié en ce qu'on avait substitué une composition contenant uniquement les trois acides aminés cationiques (Arg - Lys - His) à la composition des 12 acides aminés essentiels, ont été comparées pour ce qui concerne la quantité de PRP-T non adsorbée au final. Aucune différence dans la quantité de PRP-T non adsorbé n'a été observée, ce qui montré que seuls importent les acides aminés cationiques.

## Revendications

1. Procédé de préparation d'une combinaison vaccinale liquide comprenant au moins :
- de l'oxyhydroxyde d'aluminium (AIOOH),
- un antigène de surface de l'hépatite B (HBsAg),
- un antigène de *Haemophilus influenzae* type b (Hib) constitué par du polysaccharide capsulaire conjugué à une protéine porteuse,
dans laquelle l'antigène de surface de l'hépatite B est maintenu adsorbé sur AlOOH alors que l'antigène de Hib est maintenu non adsorbé,
selon lequel :
- on procède à l'adsorption de l'antigène de surface de l'hépatite B sur AIOOH afin d'obtenir un complexe AlOOH/HBsAg,
- on mélange ledit complexe AlOOH/HBsAg avec l'antigène de Hib en présence d'acides aminés cationiques à une concentration d'au moins 100mg/l, et d'ions Phosphate à une concentration de 35 à 45 mMol/l.

2. Procédé selon la revendication 1, selon lequel on procède à l'adsorption de l'antigène HBsAg sur l'aluminium en mélangeant une suspension d'AlOOH à une suspension d'HBsAg sous agitation pendant au moins 4 heures.

3. Procédé selon la revendication 1, selon lequel on procède à l'adsorption de l'antigène HBsAg sur l'aluminium en mélangeant une suspension d'AlOOH à une suspension d'HBsAg sous agitation pendant au moins 12 heures.

4. Procédé selon la revendication 1, selon lequel on procède à l'adsorption de l'antigène HBsAg sur l'aluminium en mélangeant une suspension d'AlOOH à une suspension d'HBsAg sous agitation entre 20 et 24 heures.

5. Procédé selon une des revendications précédentes, **caractérisé en ce qu'**on ajoute les acides aminés cationiques audit complexe AlOOH/HBsAg avant le mélange avec l'antigène de Hib.

6. Procédé selon une des revendications 1 ou 2, **caractérisé en ce qu'**on ajoute les acides aminés cationiques audit antigène de Hib avant le mélange avec le complexe AlOOH/HBsAg.

7. Procédé selon une des revendications précédentes, **caractérisé en ce qu'**on ajoute les ions Phosphate au complexe AlOOH/HBsAg avant le mélange avec l'antigène de Hib.

8. Procédé selon une des revendications précédentes, **caractérisé en ce qu'**on ajuste le pH de la préparation comprenant le complexe AlOOH/HBsAg à 7.1 ± 0.1 avant le mélange avec l'antigène de Hib.

9. Procédé selon la revendication 1, **caractérisé en ce qu'**il consiste en outre à :
- préparer une composition comprenant au moins un antigène choisi parmi les antigènes de la diphtérie, du tétanos, de la polio, de la coqueluche, ainsi que l'oxyhydroxyde d'aluminium, et
- à mélanger ledit complexe AlOOH/HBsAg avec ladite composition, avant de procéder au mélange avec l'antigène de Hib.

10. Procédé selon la revendication 9, **caractérisé en ce qu'**il consiste à préparer ladite composition en ajoutant chacun des antigènes successivement à une suspension d'oxyhydroxyde d'aluminium et en agitant entre chaque ajout d'antigènes.

11. Procédé selon la revendication 1, **caractérisé en ce que** :
- on adsorbe l'HBsAg sur une quantité partielle d'AlOOH représentant le tiers de l'AlOOH total présent dans la composition finale, pendant une durée de 20 à 24 heures, afin de former un complexe AlOOH/HBsAg,
- on adsorbe en parallèle, sur une quantité complémentaire d'AlOOH, successivement : la toxine diphtérique D, la toxine tétanique T, la toxine purifiée de *Bordetella pertussis* PTxd elle-même préalablement adsorbée sur de l'AlOOH, l'haemagglutinine filamenteuse de *Bordetella pertussis* FHA elle-même préalablement adsorbée sur de l'AlOOH, puis on y ajoute des ions Phosphate, puis on y ajoute le complexe AlOOH/HBsAg,
- on ajoute à nouveau des ions Phosphate en quantité permettant d'atteindre une concentration de 40 mMol/l dans la composition finale,
- on ajoute au moins un acide aminé cationique en quantité permettant d'atteindre une concentration d'au moins 100 mg/l dans la composition finale,
- on ajuste le pH à 7.1 ± 0.1,
- on ajoute des antigènes de la polio sous forme de virus inactivés de type 1, et/ou de type 2 et/ou de type 3,
- on ajoute l'antigène Hib,
- on ajuste le pH à 7.1 ± 0.1,
- on répartit la composition obtenue en seringue ou en flacons.

12. Procédé selon une des revendications précédentes, **caractérisé en ce qu'**on prépare à l'échelle industrielle au moins 250 l de composition vaccinale.

13. Composition vaccinale obtenue selon le procédé de l'une quelconque des revendications précédentes, et comprenant au moins l'antigène de surface de l'hépatite B (HBsAg) et l'antigène de Hib qui est constitué par du Polyribosilribitolphosphate conjugué à la protéine tétanique (PRP-T)

14. Composition vaccinale selon la revendication précédente, **caractérisée en ce qu'**elle comprend en outre des antigènes de la diphtérie, du tétanos, de la polio, de la coqueluche.

15. Composition vaccinale selon une des revendications 13 ou 14, **caractérisée en ce qu'**elle comprend au moins:
- l'antigène de surface de l'hépatite B, HBsAg,
- l'antigène de la diphtérie sous forme de toxine diphtérique D,
- l'antigène du tétanos sous forme de toxine tétanique T,
- les antigènes de la coqueluche sous forme de Toxine Purifiée (PTxd) et d'Haemagglutinine Filamenteuse (FHA),
- l'antigène de *Haemophilus influenzae* type b, sous forme de polyribosylribitolphosphate conjugué à la protéine tétanique (PRP-T),
- les antigènes de la polio sous forme de virus inactivés choisis parmi les types 1, 2 et 3.

16. Composition vaccinale selon la revendication 14, **caractérisée en ce qu'**elle comprend au moins :
- de 10 à 30 µg d'HBsAg/ml;
- de 40 à 80 Lf de D/ml;
- de 10 à 50 Lf de T/ml;
- de 40 à 60 µg de FHA/ml;
- de 40 à 60 µg de PTxd/ml;
- de 2 à 60 µg de PRP/ml sous forme de conjugué PRP-T ;
- de 1 à 2 mg d'AlOOH/ml ;
- de 35 à 45 mMol/l d'ions phosphate ;
- de 100 à 1000 mg /l d'acides aminés cationiques,
- les types 1, 2 et 3 du virus polio sous forme inactivée en quantité respective de 80, 16 et 64 DU /ml.

17. Composition vaccinale selon la revendication 16, **caractérisée en ce qu'**elle comprend au moins :
- 20 µg/ml d'HBsAg,
- 50 à 70 Lf/ml de D,
- 10 à 30 Lf/ml de T,
- 50 µg/ml de FHA,
- 50 µg/ml de PTxd,
- 20-24 µg/ml de PRP sous forme de conjugué PRP-T,
- 1.2 mg/ml d'AlOOH,
- De 38 à 42 mMol/l d'ions phosphate,
- De 400 à 800 mg/l d'acides aminés cationiques,
- Les types 1,2 et 3 du virus polio sous forme inactivée en quantité respective de 80, 16 et 64 DU/ml.

## Patentansprüche

1. Verfahren zur Herstellung einer flüssigen Impfstoffkombination, die mindestens Folgendes umfasst:
- Aluminiumoxyhydroxid (AlOOH),
- ein Hepatitis-B-Oberflächenantigen (HBsAg),
- ein *Haemophilus influenzae*-Typ-b(Hib)-Antigen, das aus einem Kapselpolysaccharid, das mit einem Trägerprotein konjugiert ist, besteht,
in der das Hepatitis-B-Oberflächenantigen auf AlOOH adsorbiert gehalten wird, während das Hib-Antigen nichtadsorbiert gehalten wird,
bei dem:
- man das Hepatitis-B-Oberflächenantigen auf das AlOOH adsorbiert, um zu einem AlOOH/HBsAg-Komplex zu gelangen,
- man den AlOOH/HBsAg-Komplex in Gegenwart von kationischen Aminosäuren in einer Konzentration von mindestens 100 mg/l und von Phosphationen in einer Konzentration von 35 bis 45 mmol/l mit dem Hib-Antigen mischt.

2. Verfahren nach Anspruch 1, bei dem man das HBsAg-Antigen dadurch auf das Aluminium adsorbiert, dass man eine AlOOH-Suspension mindestens 4 Stunden lang unter Rühren mit einer HBsAg-Suspension mischt.

3. Verfahren nach Anspruch 1, bei dem man das HBsAg-Antigen dadurch auf das Aluminium adsorbiert, dass man eine AlOOH-Suspension mindestens 12 Stunden lang unter Rühren mit einer HBsAg-Suspension mischt.

4. Verfahren nach Anspruch 1, bei dem man das HBsAg-Antigen dadurch auf das Aluminium adsorbiert, dass man eine AlOOH-Suspension zwischen 20 und 24 Stunden lang unter Rühren mit einer HBsAg-Suspension mischt.

5. Verfahren nach einem der vorhergehenden Ansprüche, **dadurch gekennzeichnet, dass** man die kationischen Aminosäuren dem AlOOH/HBsAg-Komplex vor dem Mischen mit dem Hib-Antigen zugibt.

6. Verfahren nach einem der Ansprüche 1 oder 2, **dadurch gekennzeichnet, dass** man die kationischen Aminosäuren dem Hib-Antigen vor dem Mischen mit dem AlOOH/HBsAg-Komplex zugibt.

7. Verfahren nach einem der vorhergehenden Ansprüche, **dadurch gekennzeichnet, dass** man die Phosphationen dem AlOOH/HBsAg-Komplex vor dem Mischen mit dem Hib-Antigen zugibt.

8. Verfahren nach einem der vorhergehenden Ansprüche, **dadurch gekennzeichnet, dass** man den pH-Wert des Präparats, das den AlOOH/HBsAg-Komplex umfasst, vor dem Mischen mit dem Hib-Antigen auf 7,1 ± 0,1 einstellt.

9. Verfahren nach Anspruch 1, **dadurch gekennzeichnet, dass** es weiterhin aus Folgendem besteht:
- Herstellen einer Zusammensetzung, die mindestens ein Antigen, ausgewählt aus den Diphterie-, Tetanus-, Polio-, Keuchhustenantigenen, sowie Aluminiumoxyhydroxid umfasst, und
- Mischen des AlOOH/HBsAg-Komplexes mit der Zusammensetzung, bevor man mit dem Hib-Antigen mischt.

10. Verfahren nach Anspruch 9, **dadurch gekennzeichnet, dass** es darin besteht, dass man die Zusammensetzung dadurch herstellt, dass man jedes der Antigene nacheinander zu einer Aluminiumoxyhydroxidsuspension zugibt und zwischen jeder Antigenzugabe schüttelt.

11. Verfahren nach Anspruch 1, **dadurch gekennzeichnet, dass**:
- man das HBsAg über einen Zeitraum von 20 bis 24 Stunden auf eine AlOOH-Teilmenge, die ein Drittel des Gesamt-AlOOH, das in der Endzusammensetzung vorhanden ist, adsorbiert, um einen AlOOH/HBsAg-Komplex zu bilden,
- man parallel dazu nacheinander Folgendes auf eine AlOOH-Komplementärmenge adsorbiert: das Diphterie-D-Toxin, das Tetanus-T-Toxin, das aus *Bordetella pertussis* PTxd aufgereinigte Toxin, das selbst wiederum zuvor auf AlOOH adsorbiert worden ist, das Fadenhämagglutinin von *Bordetella pertussis* FHA, das selbst wiederum zuvor auf AlOOH adsorbiert worden ist, dass man dann Phosphationen dazugibt und man dann den AlOOH/HBsAg-Komplex dazugibt,
- man nochmals Phosphationen zugibt, und zwar in einer Menge, die es ermöglicht, in der Endzusammensetzung zu einer Konzentration von 40 mmol/l zu gelangen,
- man mindestens eine kationische Aminosäure zugibt, und zwar in einer Menge, die es gestattet, zu einer Konzentration von mindestens 100 mg/l in der Endzusammensetzung zu gelangen,
- man den pH-Wert auf 7,1 ± 0,1 einstellt,
- man Polioantigene in Form von inaktivierten Viren des Typs 1 und/oder des Typs 2 und/oder des Typs 3 zugibt,
- man das Hib-Antigen zugibt,
- man den pH-Wert auf 7,1 ± 0,1 einstellt,
- man die erhaltene Zusammensetzung in Spritzen oder Vials abfüllt.

12. Verfahren nach einem der vorhergehenden Ansprüche, **dadurch gekennzeichnet, dass** man im Industriemaßstab mindestens 250 l Impfstoffzusammensetzung herstellt.

13. Impfstoffzusammensetzung, die nach dem Verfahren nach einem der vorhergehenden Ansprüche erhalten wurde und die mindestens das Hepatitis-B-Oberflächenantigen (HBsAg) und das Hib-Antigen, das aus an das Tetanusprotein konjugiertem Polyribosylribitolphosphat (PRP-T) besteht, umfasst.

14. Impfstoffzusammensetzung nach dem vorhergehenden Anspruch, **dadurch gekennzeichnet, dass** sie weiterhin Diphterie-, Tetanus-, Polio-, Keuchhustenantigene umfasst.

15. Impfstoffzusammensetzung nach einem der Ansprüche 13 oder 14, **dadurch gekennzeichnet, dass** sie mindestens Folgendes umfasst:
- das Hepatitis-B-Oberflächenantigen, HBsAg,
- das Diphtherieantigen in Form des Diphtherie-D-Toxins,
- das Tetanusantigen in Form des Tetanus-T-Toxins,
- die Antigene des Keuchhustens in Form von aufgereinigtem Toxin (PTxd) und von Fadenhämagglutinin (FHA),
- das *Haemophilus* influenzae-Typ-b-Antigen in Form des an das Tetanusprotein konjugierten Polyribosylribitolphosphats (PRP-T),
- die Polioantigene in Form von inaktivierten Viren, ausgewählt aus den Typen 1, 2 und 3.

16. Impfstoffzusammensetzung nach Anspruch 14, **dadurch gekennzeichnet, dass** sie mindestens Folgendes umfasst:
- 10 bis 30 µg HBsAg/ml;
- 40 bis 80 Lf D/ml;
- 10 bis 50Lf T/ml;
- 40 bis 60 µg FHA/ml;
- 40 bis 60 µg PTxd/ml;
- 2 bis 60 µg PRP/ml in Form des Konjugats PRP-T;
- 1 bis 2 mg AlOOH/ml;
- 35 bis 45 mmol/l Phosphationen;
- 100 bis 1000 mg/l kationische Aminosäuren,
- Typ 1, Typ 2 und Typ 3 des Poliovirus in inaktivierter Form in einer jeweiligen Menge von 80, 16 bzw. 64 DU/ml.

17. Impfstoffzusammensetzung nach Anspruch 16, **dadurch gekennzeichnet, dass** sie mindestens Folgendes umfasst:
- 20 µg/ml HBsAg,
- 50 bis 70 Lf/ml D
- 10 bis 30 Lf/ml T,
- 50 µg/ml FHA,
- 50 µg/ml PTxd,
- 20-24 µg/ml PRP in Form des Konjugats PRP-T,
- 1,2 mg/ml AlOOH,
- 38 bis 42 mmol/l Phosphationen,
- 400 bis 800 mg/l kationische Aminosäuren,
- Typ 1, Typ 2 und Typ 3 des Poliovirus in inaktivierter Form in einer jeweiligen Menge von 80, 16 bzw. 64 DU/ml.

## Claims

1. Method for preparing a liquid vaccine combination comprising at least:
- aluminium oxide hydroxide (A100H),
- one hepatitis B surface antigen (HBsAg),
- one *Haemophilus influenzae* type b (Hib) antigen consisting of capsular polysaccharide conjugated to a carrier protein,
in which the hepatitis B surface antigen is kept adsorbed on A100H, whereas the Hib antigen is kept nonadsorbed,
wherein:
- the hepatitis B surface antigen is adsorbed onto A100H in order to obtain an AlOOH/HBsAg complex,
- said AlOOH/HBsAg complex is mixed with the Hib antigen in the presence of cationic amino acids at a concentration of at least 100 mg/l and of phosphate ions at a concentration of 35 to 45 mMol/l.

2. Method according to Claim 1, wherein the HBsAg antigen is adsorbed onto the aluminium by mixing a suspension of A100H with a suspension of HBsAg with stirring for at least 4 hours.

3. Method according to Claim 1, wherein the HBsAg antigen is adsorbed onto the aluminium by mixing a suspension of AlOOH with a suspension of HBsAg with stirring for at least 12 hours.

4. Method according to Claim 1, wherein the HBsAg antigen is adsorbed onto the aluminium by mixing a suspension of A100H with a suspension of HBsAg with stirring for at least between 20 and 24 hours.

5. Method according to one of the preceding claims, **characterized in that** the cationic amino acids are added to said AlOOH/HBsAg complex before the mixing with the Hib antigen.

6. Method according to either of Claims 1 and 2, **characterized in that** the cationic amino acids are added to said Hib antigen before the mixing with the AlOOH/HBsAg complex.

7. Method according to one of the preceding claims, **characterized in that** the phosphate ions are added to said AlOOH/HBsAg complex before the mixing with the Hib antigen.

8. Method according to one of the preceding claims, **characterized in that** the pH of the preparation comprising the AlOOH/HBsAg complex is adjusted to 7.1 ± 0.1 before the mixing with the Hib antigen.

9. Method according to Claim 1, **characterized in that** it also consists in:
- preparing a composition comprising at least one antigen chosen from diphtheria, tetanus, polio and whooping cough antigens, and also aluminium oxide hydroxide, and
- mixing said AlOOH/HBsAg complex with said composition, before carrying out the mixing with the Hib antigen.

10. Method according to Claim 9, **characterized in that** it consists in preparing said composition by adding each of the antigens successively to a suspension of aluminium oxide hydroxide and by stirring between each addition of antigens.

11. Method according to Claim 1, **characterized in that**:
- the HBsAg is adsorbed onto a partial amount of AlOOH representing one third of the total AlOOH present in the final composition, for a period of 20 to 24 hours, in order to form an AlOOH/HBsAg complex,
- in parallel, the following are successively adsorbed onto an additional amount of AlOOH: diphtheria toxin D, tetanus toxin T, *Bordetella pertussis* purified toxin PTxd, itself preadsorbed onto AlOOH, and *Bordetella pertussis* filamentous hemagglutinin FHA, itself preadsorbed onto A100H, then phosphate ions are added thereto, then the AlOOH/HBsAg complex is added thereto,
- phosphate ions are again added in an amount which makes it possible to achieve a concentration of 40 mMol/l in the final composition,
- at least one cationic amino acid is added in an amount which makes it possible to achieve a concentration of at least 100 mg/l in the final composition,
- the pH is adjusted to 7.1 ± 0.1,
- polio antigens in the form of inactivated type 1 and/or type 2 and/or type 3 viruses are added,
- the Hib antigen is added,
- the pH is adjusted to 7.1 ± 0.1,
- the composition obtained is distributed into syringes or into bottles.

12. Method according to one of the preceding claims, **characterized in that** at least 250 1 of vaccine composition are prepared on an industrial scale.

13. Vaccine composition obtained according to the method of any one of the preceding claims, and comprising at least the hepatitis B surface antigen (HBsAg) and the Hib antigen which consists of polyribosylribitol phosphate conjugated to the tetanus protein (PRP-T).

14. Vaccine composition according to the preceding claim, **characterized in that** it also comprises diphtheria, tetanus, polio and whooping cough antigens.

15. Vaccine composition according to either of Claims 13 and 14, **characterized in that** it comprises at least:
- the hepatitis B surface antigen, HBsAg,
- the diphtheria antigen in the form of diphtheria toxin D,
- the tetanus antigen in the form of tetanus toxin T,
- the whooping cough antigens in the form of Purified Toxin (PTxd) and of Filamentous Hemagglutinin (FHA),
- the *Haemophilus influenzae* type b antigen, in the form of polyribosylribitol phosphate conjugated to the tetanus protein (PRP-T),
- the polio antigens in the form of inactivated viruses chosen from types 1, 2 and 3.

16. Vaccine composition according to Claim 14, **characterized in that** it comprises at least:
- from 10 to 30 µg of HBsAg/ml;
- from 40 to 80 Lf of D/ml;
- from 10 to 50 Lf of T/ml;
- from 40 to 60 µg of FHA /ml;
- from 40 to 60 µg of PTxd/ml;
- from 2 to 60 µg of PRP/ml in PRP-T conjugate form;
- from 1 to 2 mg of AlOOH/ml;
- from 35 to 45 mMol/l of phosphate ions ;
- from 100 to 1000 mg/l of cationic amino acids;
- poliovirus types 1, 2 and 3 in inactivated form, in a respective amount of 80, 16 and 64 DU/ml.

17. Vaccine composition according to Claim 16, **characterized in that** it comprises at least:
- 20 µg/ml of HBsAg,
- from 50 to 70 Lf /ml of D,
- from 10 to 30 Lf /ml of T,
- 50 µg/ml of FHA,
- 50 µg/ml of PTxd,
- from 20-24 µg/ml of PRP in PRP-T conjugate form,
- 1.2 mg/ml of A100H,
- from 38 to 42 mMol/l of phosphate ions,
- from 400 to 800 mg/l of cationic amino acids,
- poliovirus types 1, 2 and 3 in inactivated form, in a respective amount of 80, 16 and 64 DU/ml.
